# EUROPEAN PATENT SPECIFICATION

(11) **EP 3 822 625 B1**
(45) Date of publication and mention of the grant of the patent: **27.09.2023**
(21) Application number: 18925936.9
(22) Date of filing: 13.07.2018
(51) Int. Cl.: G01N 27/327, G01N 33/66, G06N 3/044, G06N 3/045, G06N 3/02

(54) **METHOD FOR MEASURING CONCENTRATION OF A BIOMETRIC MEASUREMENT OBJECT BY USING ARTIFICIAL INTELLIGENCE DEEP LEARNING**
VERFAHREN ZUR KONZENTRATIONSMESSUNG EINES BIOMETRISCHEN MESSOBJEKTS UNTER VERWENDUNG VON TIEFENLERNEN MIT KÜNSTLICHER INTELLIGENZ
PROCÉDÉ DE MESURE DE LA CONCENTRATION D'UN OBJET DE MESURE BIOMÉTRIQUE À L'AIDE D'UN APPRENTISSAGE PROFOND À INTELLIGENCE ARTIFICIELLE

(30) Priority: 11.07.2018 KR 20180080372
(43) Date of publication of application: 19.05.2021
(73) Proprietor: i-Sens, Inc., Seoul 06646 (KR)
(72) Inventor: LEE, Seok-Won, Seongnam-si, Gyeonggi-do 13554 (KR); KANG, Byeong Keun, Seoul 06113 (KR); LEE, Myeongho, Seoul 04103 (KR); NAM, Hak Hyun, Seoul 01227 (KR); LEE, Hyeonseok, Busan 47826 (KR)
(74) Representative: Arnold & Siedsma
(86) International application number: PCT/KR2018/007934
(87) International publication number: WO 2020/013361

(56) References cited:
- EP-A1- 2 998 731
- KR-A- 20090 033 065
- KR-A- 20130 131 117
- KR-A- 20160 032 974
- KR-A- 20160 096 460
- US-A1- 2008 102 441
- HUYNH HIEU TRUNG ET AL: "Accuracy Improvement for Glucose Measurement in Handheld Devices by Using Neural Networks", 1 November 2017 (2017-11-01), ICIAP: INTERNATIONAL CONFERENCE ON IMAGE ANALYSIS AND PROCESSING, 17TH INTERNATIONAL CONFERENCE, NAPLES, ITALY, SEPTEMBER 9-13, 2013. PROCEEDINGS; [LECTURE NOTES IN COMPUTER SCIENCE; LECT.NOTES COMPUTER], SPRINGER, BERLIN, HEIDELBERG, PAGE(S) 299 - 3, XP047455019, ISBN: 978-3-642-17318-9 [retrieved on 2017-11-01] * Title * * section 2.1, 2.2, 4 * * page 303, lines 11-12 * * page 304, line 2 *
- HIEU TRUNG HUYNH ET AL: "Hematocrit estimation from compact single hidden layer feedforward neural networks trained by evolutionary algorithm", EVOLUTIONARY COMPUTATION, 2008. CEC 2008. (IEEE WORLD CONGRESS ON COMPUTATIONAL INTELLIGENCE). IEEE CONGRESS ON, IEEE, PISCATAWAY, NJ, USA, 1 June 2008 (2008-06-01), pages 2962-2966, XP031325538, ISBN: 978-1-4244-1822-0

## Description

The present invention relates to an analyte concentration measurement method using artificial intelligence deep learning.

In general, one of the most important criteria for in-vitro diagnosis products is the accuracy of the measurement results.

In such products, when an analyte is present, a detection sensor outputs a signal, and an analyzer recognizes it and applies a predetermined calibration curve or algorithm to send quantitative or qualitative results.

The accuracy of the measurement result may be influenced by an interference action caused by various variables such as an interferent, an external environment, and sample properties.

In the case of an electrochemical sensor utilized for blood glucose measurement, an output electrical signal may be influenced due to a change in a diffusion coefficient toward an electrode or a reaction rate at an electrode surface due to a change in blood properties such as presence of a material other than an analyte oxidized at the electrode surface or viscosity.

In order to minimize or eliminate this effect, a method of estimating and reflecting a degree of interference of a measured object by sending various types of input signals to the sensor has been used.

For example, as disclosed in Korean Patent Registration No. 1666978, in a method for measuring concentration of an analyte in a biological sample, a redox enzyme and an electron transfer medium that can catalyze a redox reaction of the analyte were fixed, a liquid biological sample was injected into a sample cell equipped with a working electrode and an auxiliary electrode, a first sensitive current was obtained at a feature point of at least one point in time by initiating the redox reaction of the analyte and applying a constant DC voltage to the working electrode such that an electron transfer reaction can proceed, a second sensitive current was obtained at least two times or more by applying a A-stepladder-type perturbation potential after a constant DC voltage was applied, a predetermined feature was calculated from the first sensitive current or the second sensitive current, and the concentration of the analyte was calculated by using a calibration formula consisting of at least one feature function to minimize the influence of at least one interfering substance in the biological sample, so as to measure the concentration of the analyte in the biological sample.

That is, the degree of interference was estimated by using a mathematical method such as Multiple Linear Regression using parameter values derived from a predetermined signal, and the measured value was determined.

A drawback of this method is to output an unexpected and inaccurate result when it cannot distinguish or detect a given rule of previously extracted features or input command.

Although most features are focused on excluding an influence of hematocrit, when at least two interfering species other than hematocrit are combined in combination to affect the concentration of the bioanalyte, the features and a calibration equation using these features have to be corrected, but otherwise, it may cause fatal problems.

In addition to the hematocrit, even when at least two interfering species are combined to affect the concentration of the bioanalyte, there was a problem in that a biometric strip was abused because the abnormality was not known.

Furthermore, for external environmental factors, for example, a value of the temperature feature is a value obtained through a temperature sensor attached to a meter, and when there is a sudden change in the surrounding environment, it is difficult to measure the correct temperature immediately and there are many cases where time is required to achieve temperature equilibrium.

Therefore, most meters require a user to wait a certain amount of time to equilibrate the temperature in order to use the meter when the user is in a suddenly changed environment (ex. enters a warm house from outside on a cold winter day).

A method for measuring a concentration of an analyte in a bio-sample using an electrochemical bio-sensor is known from EP2998731A1. This method is characterized by a step of obtaining predetermined features from induced currents obtained by applying a DC voltage according to chronoamperometry in which, after a whole blood sample is injected to the electrochemical bio-sensor, a concentration of an analyte is obtained from an induced current obtained by applying a DC voltage for a certain time and from all induced currents obtained by further applying several step-ladder perturbation potentials for a short time subsequent to the DC voltage for the certain time, and is also characterized by minimization of a measurement error caused by a hindering material by forming a calibration equation by combining the at least one feature in a function and optimizing various conditions of the bio-sample through multivariable analysis. With this configuration, a perturbation potential application method added to a conventional measurement method can maintain a bio-sensor and a measuring apparatus already used, a line used in the measuring apparatus, and calibration of amperometry as they are, improve accuracy in measurement by effectively minimizing a matrix interference effect of a background material in a bio-sample, particularly an inaccuracy caused by a change in hematocrit, and also remarkably improve accuracy in measurement by simply upgrading a measurement program of a conventional measuring apparatus.

The present invention has been made in an effort to provide an analyte concentration measurement method using artificial intelligence deep learning, capable of extracting useful features that are not known in advance by humans through deep learning using artificial neural networks by databasing input signals obtained during the measurement time from samples with information (labels) and estimating a result value by applying an algorithm obtained through learning in this way, compared with conventional measurement techniques that are applied by devising formulas or methods that directly extract features for a long time by experts in related fields in order to extract effective features.

The present invention has been made in an effort to provide an analyte concentration measurement method using artificial intelligence deep learning to obtain features that may be classifiers for classifying groups without the need to search for a separate feature each time, compared with conventional measurement techniques that can be a regression model that estimates a specific value, which takes a lot of time and effort to find a feature each time at which a shape of an input waveform entering a sensor including a sample changes.

The present invention has been made in an effort to provide an analyte concentration measurement method using artificial intelligence deep learning, capable of automatically calibrating features and calibration formulas using these features when at least two interfering species other than hematocrit are combined in combination to affect the concentration of the bioanalyte.

The present invention has been made in an effort to provide an analyte concentration measurement method using artificial intelligence deep learning, capable of announcing special abnormalities when at least two interfering species other than hematocrit are combined in combination to affect the concentration of the bioanalyte.

In addition, the present invention has been made in an effort to provide an analyte concentration measurement method using artificial intelligence deep learning, capable of providing constant accuracy even when measuring the concentration of a bioanalyte without waiting for a certain period of time for stabilization of changes in an external environment against sudden changes in the external environment.

An exemplary embodiment of the present invention provides a method for measuring concentration of an analyte material in a biological sample, using artificial intelligence, including: injecting a liquid biological sample into a sample cell having a working electrode and an auxiliary electrode, in which an electron transport medium and a redox enzyme which is capable of catalyzing a redox reaction of an analyte are fixed; obtaining a first sensitive current at a characteristic point of at least one point of time by applying a constant DC voltage to the working electrode to initiate the redox reaction of the analyte and to proceed with an electron transfer reaction; obtaining a second sensitive current at two or more points of time by applying a A-stepladder-type perturbation potential after applying the constant DC voltage; calculating a predetermined feature from the first sensitive current or the second sensitive current; and correcting concentration of the analyte by using an calibration formula composed of at least one feature function by artificial intelligence learning such that an influence of at least one interfering substance in the biological sample is minimized.

In accordance with an analyte concentration measurement method utilizing artificial intelligence deep learning according to an exemplary embodiment of the present invention, an object of the present invention capable of extracting useful features that are not known in advance by humans through deep learning using artificial neural networks by databasing input signals obtained during the measurement time from samples with information (labels) and estimating a result value by applying an algorithm obtained through learning in this way, compared with conventional measurement techniques that are applied by devising formulas or methods that directly extract features for a long time by experts in related fields in order to extract effective features, is to obtain features that may serve as a classifier for classifying a group without the need to search for a separate feature each time, compared with conventional measurement techniques, which were to devise and apply a formula or method that directly extracts features for a long time by experts in related fields in order to extract effective features, can be a regression model that estimates a specific value, which takes a lot of time and effort to find a feature each time at which a shape of an input waveform entering the sensor containing the sample changes.

In addition, in accordance with an analyte concentration measurement method utilizing artificial intelligence deep learning according to an exemplary embodiment of the present invention, it is possible to automatically correct features and calibration formulas using these features when at least two interfering species other than hematocrit are combined in combination to affect the concentration of the bioanalyte.

In accordance with an analyte concentration measurement method utilizing artificial intelligence deep learning according to an exemplary embodiment of the present invention, it is possible to announce special abnormalities when at least two interfering species other than hematocrit are combined in combination to affect the concentration of the bioanalyte.

In accordance with an analyte concentration measurement method utilizing artificial intelligence deep learning according to an exemplary embodiment of the present invention, it is possible to provide constant accuracy even when measuring the concentration of a bioanalyte without waiting for a certain period of time for stabilization of changes in an external environment against sudden changes in the external environment.
FIG. 1 illustrates an internal configuration diagram of a blood glucose measurement device using artificial intelligence deep learning according to an example of the present invention.
FIG. 2 illustrates a flowchart of a blood glucose measurement method using artificial intelligence deep learning according to an exemplary embodiment of the present invention.
FIG. 3 illustrates a detailed view of an artificial intelligence learning calibrator in an internal configuration diagram of a blood glucose measurement device using artificial intelligence deep learning according to an example of the present invention.
FIG. 4 illustrates a graph showing a A-stepladder-type perturbation potential used in a blood glucose measurement method using artificial intelligence deep learning and a corresponding sensitive current according to an exemplary embodiment of the present invention.
FIG. 5 illustrates an exemplary diagram showing results of a regression model in which blood glucose estimation is performed depending on a blood glucose estimating method using a conventional multiple regression method and an analyte concentration measurement method utilizing a deep learning method using an artificial neural network according to an exemplary embodiment of the present invention.
FIG. 6 illustrates a graph showing robustness of an algorithm according to noise when a random noise of a certain magnitude is applied to an input signal depending on an analyte concentration measurement method utilizing artificial intelligence deep learning according to an exemplary embodiment of the present invention.
FIG. 7 illustrates an exemplary diagram showing results of a regression model in which blood glucose estimation is performed when a temperature feature is not included and when the temperature feature is included depending on an analyte concentration measurement method utilizing artificial intelligence deep learning according to an exemplary embodiment of the present invention.
FIG. 8A and FIG. 8B respectively illustrate graphs showing robustness of an algorithm when an ambient temperature is higher than a room temperature and lower than the room temperature depending on an analyte concentration measurement method utilizing artificial intelligence deep learning according to an exemplary embodiment of the present invention.

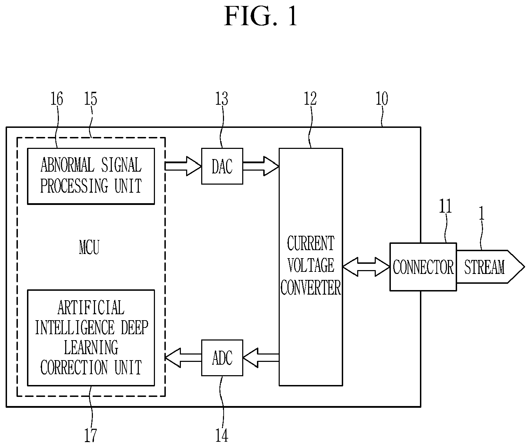

Hereinafter, an analyte concentration measurement method utilizing a deep learning method using an artificial neural network according to an exemplary embodiment of the present invention will be described in detail with reference to FIG. 1 to FIG. 6.

Hereinafter, a blood glucose measurement method utilizing artificial intelligence deep learning according to an exemplary embodiment of the present invention will be described with reference to FIG. 1 to FIG. 4.

FIG. 1 illustrates an internal configuration diagram of a blood glucose measurement device using artificial intelligence deep learning according to an example of the present invention.

As illustrated in FIG. 1, the blood glucose measurement device 10 utilizing artificial intelligence deep learning according to an exemplary embodiment of the present invention may provide blood glucose measurement values depending on an optimized artificial intelligence-based deep learning algorithm for blood glucose estimation capable of improving algorithm accuracy, precision, and interference correction performance including hematocrit beyond a conventional multiple linear regression method according to a blood glucose measurement method using the artificial intelligence deep learning by applying a A-stepladder-type perturbation potential after applying a certain voltage while maintaining a structure of an existing electrochemical biosensor, i.e., a pair of working electrodes and auxiliary electrodes of a strip 1.

The blood glucose measurement device 10 using artificial intelligence deep learning according to an exemplary embodiment of the present invention may also efficiently calculate an artificial intelligence-based deep learning algorithm for estimating blood glucose that is optimized even in limited hardware, so that the calculation time may be within 8 s.

The blood glucose measurement device 10 using artificial intelligence deep learning according to an exemplary embodiment of the present invention may be configured such that, when the electrochemical biosensor strip 1 is mounted on a connector 11, the connector 11 is electrically connected to a current-voltage converter 12, and a microcontroller (MCU) 15 may apply a constant voltage through a digital-analog converter circuit (DAC) 13 and a A-stepladder-type perturbation potential to the working electrode of the strip 1.

To this end, firmware of the blood glucose measurement device 10 using artificial intelligence deep learning according to an exemplary embodiment of the present invention first stores a constant capable of generating a predetermined triangular wave circulating voltage in a memory, records a predetermined constant to the register of the DAC 13 when applying a constant voltage, and increases or decreases the constant stored in the memory at a predetermined period of time to record it in a register of the DAC 13 when applying the A-stepladder-type perturbation potential. However, the waveform of the A-stepladder-type perturbation potential exemplified herein is merely an example, but the present invention is not limited to this example, and includes all waveforms of a circulating voltage that are natural to the workers in the project.

The microcontroller 15 applies the corresponding voltage between two electrodes of the strip depending on the constant recorded in the register of the DAC 13.

When the constant voltage is applied and the A-stepladder-type perturbation potential is applied, a response current measured through the strip 1 may be measured directly by an analog-digital converter circuit (ADC) 14 through the connector 11 and the current-voltage converter 12.

On the other hand, the blood glucose measurement device 10 using artificial intelligence deep learning according to an exemplary embodiment of the present invention further includes an abnormal signal processing unit 16 and an artificial intelligence deep learning algorithm calculation unit 17, and when the strip 1 is poorly connected to the connector 11 or an abnormal signal due to abnormal blood injection or hardware is detected, the abnormal signal processing unit 16 may notify this through an alarm, a display, etc., and may prevent the artificial intelligence deep learning correction unit 17 from performing unnecessary calculations.

The artificial intelligence deep learning algorithm calculation unit 17 may obtain a blood glucose measurement value from a response current measured through the strip 1 by an optimized artificial intelligence deep learning blood glucose measurement algorithm within 8 s.

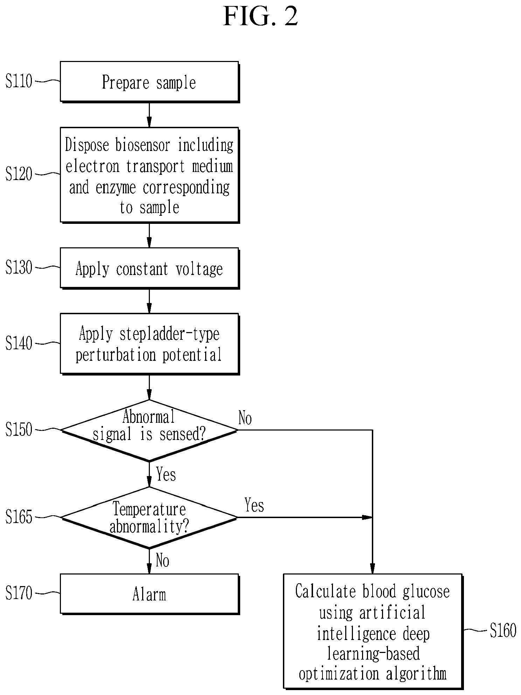

Hereinafter, a blood glucose measurement method utilizing artificial intelligence deep learning according to an exemplary embodiment of the present invention will be described with reference to FIG. 2.

FIG. 2 illustrates a flowchart of a blood glucose measurement method using artificial intelligence deep learning according to an exemplary embodiment of the present invention.

Referring to FIG. 2, the blood glucose measurement method the using artificial intelligence deep learning according to the exemplary embodiment of the present invention includes: preparing a sample (S110); loading, e.g., an electrochemical face-to-face biosensor strip 1 in a biometric information measurement device 10 (S120); applying a constant voltage through a current-voltage converter 12 at a moment when blood soaks a working electrode and an auxiliary electrode of the electrochemical biosensor 1, as the biosensor strip 1 contacts the sample (blood); and continuously applying a A-stepladder-type perturbation potential at an end of an applied voltage after the constant voltage is applied (S140).

The abnormal signal processing unit 16 determines whether a sensitive current is an abnormal signal (S 150), and when it is determined as the abnormal signal, this may be notified through an alarm or display (S151).

In this case, when it is determined as the abnormal signal because an ambient temperature or a temperature of the strip or blood is too much higher or lower than an indoor temperature, calculation of a blood glucose value using an artificial intelligence deep learning-based optimization algorithm may be performed without a waiting time.

In addition, when it is determined that it is not the abnormal signal, a blood glucose measurement value may be obtained from a response current measured by an artificial intelligence deep learning blood glucose measurement algorithm optimized by the artificial intelligence deep learning correction unit 17.

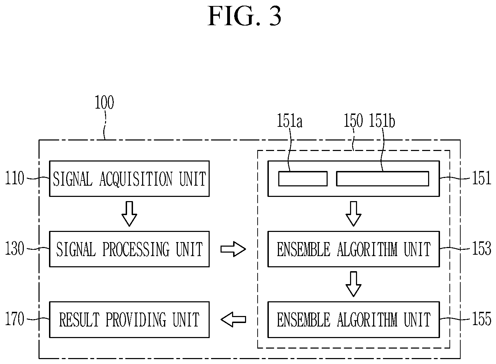

FIG. 3 illustrates a detailed view of an artificial intelligence learning calibrator in an internal configuration diagram of a blood glucose measurement device using artificial intelligence deep learning according to an exemplary embodiment of the present invention.

As illustrated in FIG. 3, the artificial intelligence learning correction unit 17 may download or store an artificial intelligence learning algorithm optimized through a wired/wireless network through software with respect to a biometric information analysis artificial intelligence-based deep learning server 100, and the biometric information analysis artificial intelligence-based deep learning server 100 may include: a signal acquisition unit 110 configured to acquire one-dimensional time series data through an electrochemical reaction that occurs by injecting blood collected through the biometric information measurement device 10 into a sensor (strip); a signal processing unit 130 configured to preprocess a signal to exclude abnormal signals due to blood injection abnormality and hardware abnormality among signals acquired from the signal acquisition unit 110 or to obtain an optimized biometric information measurement algorithm that is to be used in the biometric information measurement device 10; a biometric information measurement algorithm generating unit 150 configured to automatically extract features of the optimized biometric information measurement algorithm utilizing a deep learning artificial neural network technique by using the signal processed through the signal processing unit 130; and an optimization algorithm result providing unit 170 configured to be used in the biometric information measurement device 10.

The signal processing unit 130 may convert data into a certain size or distribution through normalization or standardization such that the biometric information measurement algorithm generation unit 150 can learn it, and the converted data may be converted into a data image by combining multi-channel data or using signal processing and data conversion such as domain conversion (e.g., time or frequency domain).

The biometric information measurement algorithm generator 150 may include: an algorithm structure unit 151 configured to form an algorithm structure for measuring blood glucose; an algorithm learning unit 153 configured to adjust variables in the algorithm to accurately predict a blood glucose value, where a predicted blood glucose value is true; and an ensemble algorithm unit 155 configured to calculate a final predicted value by combining one or more algorithms to improve accuracy and precision of blood glucose value prediction.

In addition, the algorithm structure unit 151 may include: a feature extraction unit 151a configured to extract a feature of an analyte included in the biometric information signal data preprocessed by the signal processing unit 130; and a blood glucose value predicting unit 151b configured to estimate a blood glucose value by using features obtained by using the feature extracting unit 151a.

The algorithm structure unit 151 automatically extracts features reflecting the result value to be classified or measured from the image reflecting a surrounding environment, such as the components of the analyte, the hematocrit, the temperature, and the characteristics of the interfering species, utilizing a deep learning artificial neural network technique.

The algorithm learning unit 153 derives a weight and a bias between layers of artificial neural networks of which an error of a result value is minimal through an algorithm learning process using the extracted features.

The algorithm structure unit 151, which is an artificial neural network algorithm, may be utilized as a regression model to estimate a specific value depending on a purpose, and may also be used as a classifier to classify types of analytes.

In the present specification, correcting the occurrence of measurement errors due to hematocrit during blood glucose measurement is described as an exemplary embodiment, but the concentration of various metabolites, e.g., organic or inorganic substances such as beta hydroxybutyrate (aka ketone), cholesterol, lactate, creatinine, hydrogen peroxide, alcohol, amino acids, or glutamate may be corrected in the same way by introducing a specific enzyme like a glucose test. Accordingly, the present invention can be used to quantify various metabolites by varying types of enzymes included in sample layer composition.

For example, quantification of beta hydroxybutyrate, glucose, glutamate, cholesterol, lactate, ascorbic acid, alcohol, and bilirubin may be performed by β-hydroxybutyrate dehydrogenase, glucose oxidase (GOx), glucose dehydrogenase (GDH), glutamate oxidase, glutamate dehydrogenase, cholesterol oxidase, cholesterol esterase, lactate oxidase, ascorbic acid oxidase, alcohol oxidase, alcohol dehydrogenase, bilirubin oxidase, etc.

In the biometric information measurement device 10 according to an exemplary embodiment of the present invention, a working electrode and an auxiliary electrode are provided to face each other on different planes, and a face-to-face electrochemical biosensor coated with a reagent composition including an enzyme and an electron transport medium depending on a material may be applied to the working electrode.

In addition, in the biometric information measurement device 10 according to an exemplary embodiment of the present invention, a working electrode and an auxiliary electrode may be provided on one plane, and a planar electrochemical biosensor coated with a reagent composition including an enzyme and an electron transport medium depending on a material may be applied on the working electrode.

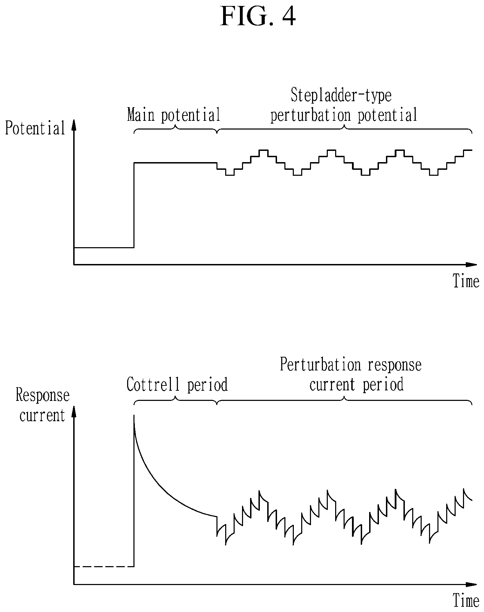

Now, a reason for using the A-stepladder-type perturbation potential will be described simply with reference to FIG. 4.

FIG. 4 illustrates a graph showing a A-stepladder-type perturbation potential used in a blood glucose measurement method using artificial intelligence deep learning and a corresponding sensitive current according to an exemplary embodiment of the present invention.

On the other hand, in accordance with the blood glucose measurement method using the artificial intelligence deep learning according to the exemplary embodiment of the present invention, as illustrated in FIG. 4, concentration of the bioanalyte is measured through the sensitive current by applying the stepladder-type perturbation potential after a constant voltage (VDC) is applied, and the application of the stepped ladder-shaped perturbation potential in this way causes an important change in the characteristics of the sensitive current, so as to eliminate or minimize an influence of an erythrocyte volume ratio or other interfering species.

Herein, the sensitive current is expressed as a first sensitive current or a second sensitive current to indicate that characteristics of the sensitive current are changed by fluctuation or perturbation and are different from each other.

A stepped ladder perturbation potential application method with periodicity that is additionally applied for a short period of time for the purpose of removing an effect of an erythrocyte volume ratio in a calibration formula after applying a constant voltage is referred to as "A-stepladder perturbation potential" or simply "stepladder potential."

Currents with different characteristics refer to currents that can be used as a variable that can effectively separate or correct an effect of an erythrocyte volume ratio because a method depends on blood glucose and the erythrocyte volume ratio (blocking substance).

A method of finding feature points in the second sensitive currents corresponding to a period during which a perturbation potential is applied and a method of creating a feature from the feature points will be described as follows.

The method below is merely an example, and may be applied by various modifications depending on a purpose of application.
1) Sensitive currents near peak and valley voltages of a specific stepped ladder
2) Curvature of a curve consisting of the sensitive currents of each step in the stepped ladder
3) Difference between a current value at the stepped ladder peak and a current at the valley
4) Sensitive currents in the stepped ladder between uphill and downhill
5) Sensitive currents at a start point and an end point of each stepped ladder cycle
6) Average value of sensitive currents obtained from stepped ladder waves
7) Values that can be obtained by expressing the current values obtained from Features 1 to 6 as mathematical functions such as four arithmetic operations, exponents, logarithms, trigonometric functions, etc.

In this way, the feature points may be found in the second sensitive currents corresponding to the period during which the perturbation potential is applied, or the current values obtained from the feature points may be made into a feature, and these may be linearly combined to apply multivariable regression analysis, so that a calibration formula that minimizes the effect of the erythrocyte volume ratio may be obtained.

The calibration formula is one of 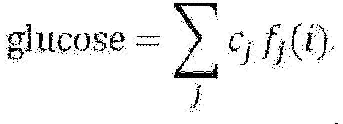 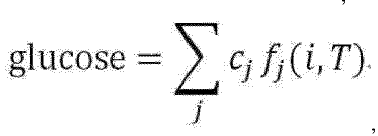 and

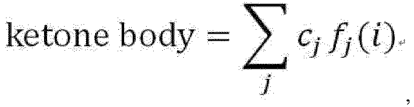

Herein, i is a current value that is greater than or equal to one obtainable from the first and second sensitive currents, and T is an independently measured temperature value.

Referring back to FIG. 3, the signal acquisition unit 110 may acquire one-dimensional time series data through an electrochemical reaction that occurs by injecting drawn blood into a sensor strip.

The signal preprocessing unit 130 may image the one-dimensional time series data as two or more-dimensional data through signal processing and data conversion, and may use it as an input signal of an artificial neural network.

The biometric information measurement algorithm generation unit 150 automatically extracts features reflecting a result value to be classified or measured from an image reflecting the component of the analyte and the surrounding environment using a deep learning artificial neural network technique.

Algorithm learning is performed using the features extracted through the algorithm learning unit 153 to derive weights and biases between layers of artificial neural networks of which errors of a result value are minimal.

An artificial neural network algorithm may be utilized as a regression model to estimate a specific value depending on a purpose, and may also be used as a classifier to classify types of analytes.

In the analyte measurement method using artificial intelligence deep learning according to the exemplary embodiment of the present invention, experiments are conducted by reflecting various factors that affect blood glucose values in order to obtain the learning data to create an algorithm of a blood glucose meter.

The main factors affecting the blood glucose values include blood glucose, hematocrit, measured temperature, partial pressure of oxygen, and the like.

Blood samples of various experimental conditions are produced, and learning data for making an algorithm is obtained through repeated measurements.

The obtained learning data, which are one-dimensional time series data, represent an electrochemical reaction of the analyte over time.

Learning data is converted to a certain scale or distribution through normalization or standardization.

The converted data can be converted into a data image by combining multi-channel data or using signal processing and data conversion such as domain conversion (e.g., time or frequency domain).

The imaged data may learn an algorithm that can output an appropriate result depending on an input using artificial neural network deep learning technique.

The artificial neural network is a method that mimics a principle of an operation of a human brain, and it is desirable to control weights between neurons in several layers in the artificial neural network.

These artificial neural networks include convolutional neural networks (CNNs), deep belief networks (DBNs), and recurrent neural networks (RNNs) depending on their structure. A difference between the artificial neural network deep learning technique and other machine learning is that features can be automatically extracted.

There are several methods for automatic feature extraction, such as restricted Boltzmann machines (RBM).

RBM may be used for unsupervised learning, and may go through an optimization process that makes the distribution of the input data and the distribution of reconstructed data that is determined (stochastic decision) depending on probability similar.

As a result, a result value of a hidden layer may be used as a feature value representing the input data.

It is possible to construct the structure of the artificial neural network by expanding the hidden layer into several layers to obtain the characteristics of each hidden layer, by repeatedly performing the above process.

A purpose of artificial neural network learning is to minimize output errors.

Methods for minimizing output errors include Levenberg-Marquardt, Gauss-Newton, Gradient descent, and the like.

Through the above optimization method, weight and bias values of the entire artificial neural network are determined.

In addition to weights and biases in artificial neural networks, an activation function also plays an important role.

The activation function determines how to receive an input signal from each neuron (node) and to send an output.

The activation function includes sigmoid, hyperbolic tangent, rectified linear unit, and the like.

The artificial neural network may be used for a classifier that classifies a type of data through a change in the activation function or structure of the output layer, or for regression that estimates a value.

For example, it may be used as a classifier that separates human blood from a control solution, and may also be used for regression analysis to estimate blood glucose values.

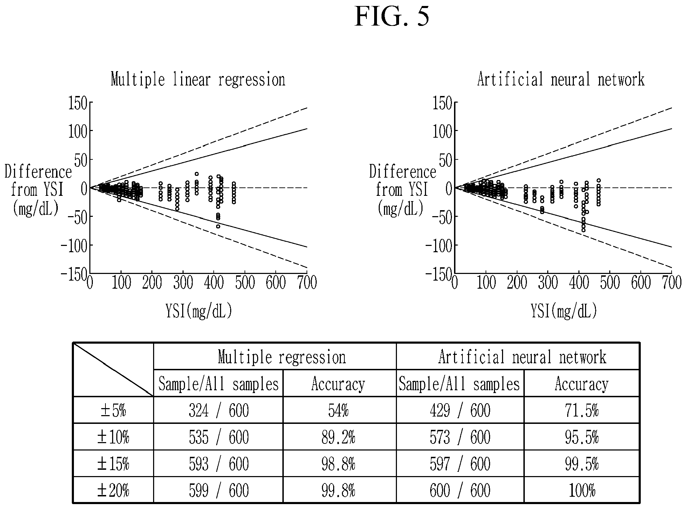

As illustrated in FIG. 5, results of a blood sugar estimation method using a conventional multiple regression method and a regression model using an artificial neural network to estimate blood glucose may be compared.

As a result of comparing an estimated blood glucose value with that of a YSI analyzer, which is a blood glucose reference measurement device, it can be seen that the estimated blood glucose value using an artificial neural network shows more accurate results than that of the conventional multiple regression method.

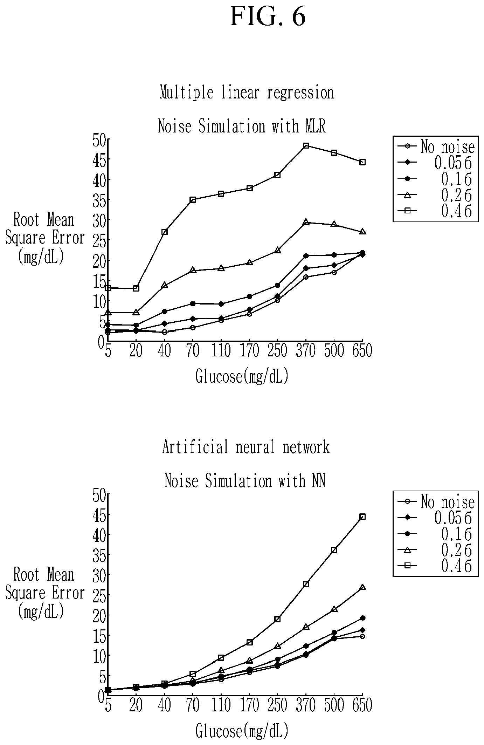

As illustrated in FIG. 6, when random noise of a predetermined magnitude is applied to an input signal, robustness of the algorithm depending on the noise may be confirmed.

Accordingly, the robustness of the algorithm was confirmed by a root mean square error of the YSI value, which is the reference value, and a measurement algorithm value.

In the case of multiple regression, it reacts sensitively to noise and the output value appears unstable regardless of blood concentration.

On the other hand, in the case of the artificial neural network, particularly at low concentration, it can be seen that it is robust against noise and the output value is more stable than with the regression method.

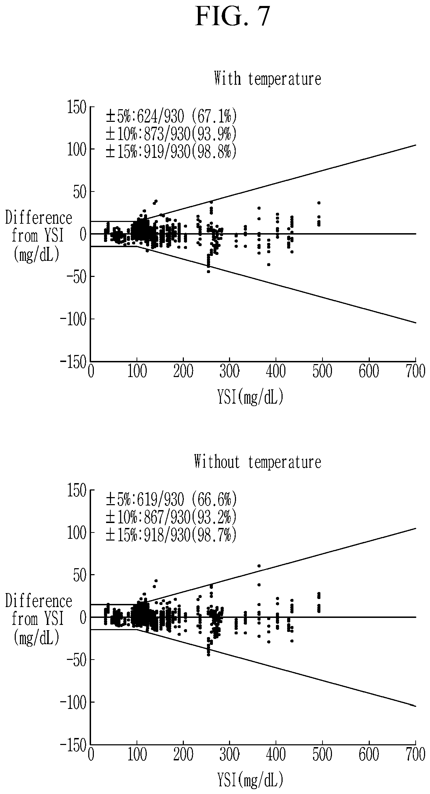

When a difference between the ambient temperature and the balancing temperature, which is one of the factors that can affect blood concentration, is large, an analyte concentration measurement method using artificial intelligence deep learning according to an exemplary embodiment of the present invention will be described by way of examples with reference to FIG. 7 to FIG. 8B.

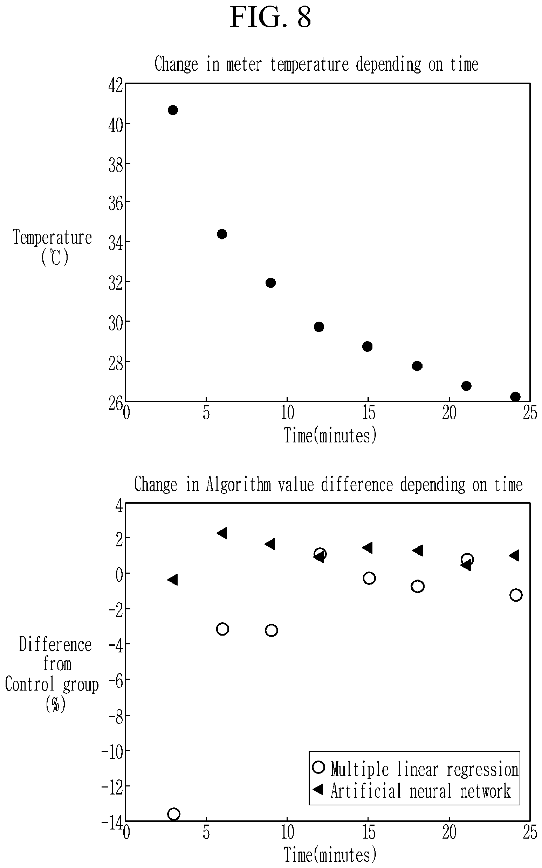

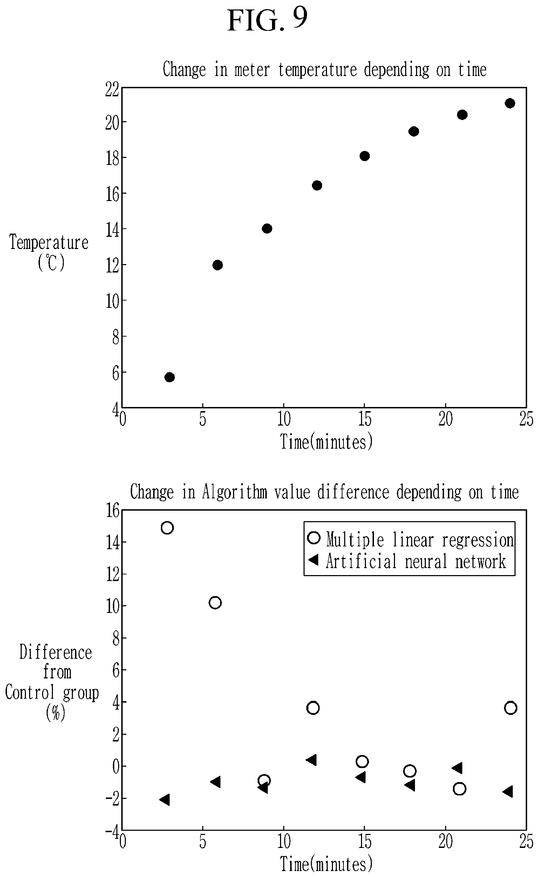

FIG. 7 illustrates an exemplary diagram showing results of a regression model in which blood glucose estimation is performed when a temperature feature is not included and when the temperature feature is included depending on an analyte concentration measurement method utilizing artificial intelligence deep learning according to an exemplary embodiment of the present invention, and FIG. 8A and FIG. 8B respectively illustrate graphs showing robustness of an algorithm when an ambient temperature is higher than room temperature and lower than room temperature depending on an analyte concentration measurement method utilizing artificial intelligence deep learning according to an exemplary embodiment of the present invention.

### (Example 1) Performance comparison depending on presence or absence of a temperature feature of a neural net model

Two identical artificial intelligence deep learning algorithm models including/without a temperature feature in the neural net algorithm of a same structure were implemented and shown in Table 1 and FIG. 7 in order to observe a performance change in a neural net algorithm depending on presence or absence of the temperature feature.

Performance comparison was confirmed through a total of 930 samples and clinical tests of a total of 9 lots.

As illustrated in FIG. 7, when a difference between YSI values, which are reference values, in the cases of including and not including the temperature feature, a case of ±5 % shows average performance accuracy of 66.6 % and 67.1 %, a case of ±10 %shows average performance accuracy of 93.2 % and 93.9 %, and a case of ±15 % shows average performance accuracy of 98.7 % and 98.8 %, so it was confirmed that there was little difference in the performance of the neural net algorithm depending on presence or absence of the temperature feature in the clinical data.

**(Table 1)**

| **Lot** | **Artificial neural network without temperature** | | | **Artificial neural network including temperature** | | |
|---|---|---|---|---|---|---|
| | **±5 %** | **±10 %** | **±15 %** | **±5 %** | **±10 %** | **±15 %** |
| **1** | **70.8** | **92.5** | **100** | **67.9** | **92.5** | **99.1** |
| **2** | **72.6** | **98.1** | **100** | **69.8** | **98.1** | **99.1** |
| **3** | **60.4** | **92.5** | **100** | **58.5** | **93.4** | **100** |
| **4** | **66.7** | **92.2** | **98** | **68.6** | **96.1** | **99** |
| 5 | 61.8 | 92.2 | 97.1 | 64.7 | 90.2 | 97.1 |
| 6 | 69.6 | 96.1 | 100 | 71.6 | 98 | 100 |
| 7 | 63.7 | 92.2 | 99 | 72.5 | 96.1 | 100 |
| 8 | 73.5 | 93.1 | 98 | 71.6 | 93.1 | 99 |
| 9 | 55.9 | 88.2 | 96.1 | 58.8 | 87.3 | 98 |
| Total | 66.6 | 93.2 | 98.7 | 67.1 | 93.9 | 98.8 |

### (Example 2) Comparison of changes in algorithm results for meter temperature differences according to rapid environmental changes

A value of the temperature feature applied to the algorithm is a value obtained through a temperature sensor attached to a meter, and when there is a sudden change in the surrounding environment, it is difficult to measure the correct temperature immediately and time is required to achieve temperature equilibrium.

Therefore, when in a rapid environment change due to these problems, e.g., when entering a warm house from outside on a cold winter day, in order to use the meter, it is required to wait a certain time for temperature equilibration, otherwise, an incorrect blood glucose value may be obtained.

In contrast, with reference to Experimental Example 1, it will be described that such a problem can be solved by a bioanalyte concentration measurement method utilizing artificial intelligence deep learning according to an exemplary embodiment of the present invention, which does not include temperature.

For this purpose, in a process of equilibrating the temperature from 43 to 23 °C and a process of equilibrating the temperature from 0 to 23 °C with a control group experimenting in a 23 °C environment after equilibrating the temperature at 23 °C, a difference in the algorithm values of experimental groups 1 and 2 according to the error of the measured temperature of the meter was compared with the case of the conventional multiple regression analysis method and the neural net algorithm without temperature was shown in Table 2 and FIG. 8A and FIG. 8B.

**(Table 2)**

| | | Multiple linear regression | | | Artificial neural network | | |
|---|---|---|---|---|---|---|---|
| **Time (minutes)** | **Temperat ure (°C)** | **Control group** | **Experimen tal group 1** | **Bias from control group (%)** | **Control group** | **Experimen tal group 1** | **Bias from control group (%)** |
| **3** | **40.7** | **126** | **110** | **-13.6** | **133** | **132** | **-0.4** |
| **6** | **34.4** | **125** | **121** | **-3.2** | **129** | **132** | **2.2** |
| **9** | **32.0** | **123** | **119** | **-3.3** | **131** | **133** | **1.6** |
| **12** | **29.8** | **122** | **123** | **1.0** | **130** | **132** | **0.9** |
| **15** | **28.8** | **122** | **121** | **-0.3** | **129** | **131** | **1.4** |
| **18** | **27.8** | **125** | **124** | **-0.8** | **129** | **130** | **1.2** |
| **21** | **26.8** | **120** | **121** | **0.7** | **129** | **129** | **0.4** |
| **24** | **26.2** | **122** | **120** | **-1.3** | **129** | **130** | **1.0** |

| | | **Multiple linear regression** | | | **Artificial neural network** | | |
|---|---|---|---|---|---|---|---|
| **Time (minutes)** | **Temperat ure (°C)** | **Control group** | **Experimen tal group 2** | **Bias from control group (%)** | **Control group** | **Experimen tal group 2** | **Bias from control group (%)** |
| **3** | **5.8** | **131** | **152** | **14.8** | **145** | **142** | **-2.1** |
| **6** | **12.0** | **127** | **141** | **10.1** | **143** | **141** | **-1.0** |
| **9** | **14.1** | **133** | **132** | **-0.9** | **142** | **140** | **-1.4** |
| **12** | **16.4** | **132** | **136** | **3.6** | **141** | **142** | **0.4** |
| **15** | **18.1** | **132** | **132** | **0.3** | **142** | **141** | **-0.7** |
| **18** | **19.4** | **129** | **129** | **-0.3** | **139** | **138** | **-1.1** |
| **21** | **20.4** | **132** | **130** | **-1.4** | **138** | **138** | **-0.1** |
| **24** | **21.1** | **131** | **135** | **3.5** | **141** | **139** | **-1.6** |

As an experiment result, 1 meter of the experimental group in the 43 °C environment shows a difference in the experimental environment of 23 °C and the measured temperature value until about 24 minutes pass. This difference with the measured temperature shows a maximum difference of 13.6 % from the control group in the multiple linear regression algorithm, and a waiting time for temperature equilibration of about 9 minutes was required. On the other hand, the artificial neural network algorithm maintained a difference of 0 to 2 % from that of the control group regardless of the difference in temperature, and was immediately available without a waiting time.

Similarly, 2 meter of the experimental group in the 58 °C environment shows a difference in the experimental environment of 23 °C and the measured temperature value until about 24 minutes pass. This difference with the measured temperature shows a maximum difference of 14.8 % from the control group in the multiple linear regression algorithm, and a waiting time for temperature equilibration of about 15 minutes was required.

On the other hand, the artificial neural network algorithm maintained a difference of 0 to 2 % from that of the control group regardless of the difference in temperature, and was immediately available without a waiting time.

Through the experimental result, it was confirmed that the neural net algorithm without temperature can obtain more accurate results for rapid environmental changes, and that users can use the product without a special waiting time.

## Claims

1. A bioanalyte concentration measurement method using artificial intelligence deep learning, the method comprising:
injecting a liquid biological sample into a sample cell equipped with a working electrode and an auxiliary electrode of an electrochemical biosensor strip loaded in a biometric information measurement device (S120), in which an electron transport medium and a redox enzyme which is capable of catalyzing a redox reaction of an analyte are fixed;
obtaining a first sensitive current at a characteristic point of at least one point of time by applying a constant DC voltage to the working electrode to initiate the redox reaction of the analyte and to proceed with an electron transfer reaction (S130);
obtaining a second sensitive current at two or more points of time by applying a A-stepladder-type perturbation potential after applying the constant DC voltage (S140);
calculating a feature representing a concentration of the analyte from the first sensitive current or the second sensitive current; and
correcting the calculated concentration of the analyte by using artificial intelligence deep learning such that an influence of at least two interfering species in the biological sample is minimized,
wherein the correcting of the calculated concentration of the analyte includes calculating a new feature representing a concentration of the analyte by reacquiring the first and second sensitive currents by artificial intelligence deep learning, and
wherein the artificial intelligence deep learning includes:
making an artificial neural network deep learning algorithm by:
obtaining blood samples of various experimental conditions, and obtaining learning data through repeated measurements,
the obtained learning data, which is one-dimensional time series data, representing an electrochemical reaction of the analyte over time;
converting the obtained learning data to a certain distribution through normalization or standardization of the learning data, the converted data being signal-processed by combining multi-channel data or performing domain conversion; and
learning the algorithm with the converted learning data, wherein the algorithm is configured to output a result depending on an input using artificial neural network deep learning technique;
wherein the artificial intelligence deep learning corrects the at least two interfering species other than hematocrit; and
wherein concentration measurement of the analyte in the biological sample is corrected by artificial intelligence deep learning without a waiting time for temperature balancing.

2. The bioanalyte concentration measurement method of claim 1, which is a method of making a feature with a feature point by selecting a feature point having a different linear dependence on the analyte and an interfering material in the first or second sensitive currents, constructing a feature from the feature point, creating a test formula composed of the above feature by the artificial intelligence learning uses the second sensitive current near a peak and valley voltage of a specific stepped ladder, curvature of the curve consisting of the sensitive currents of each step in a stepladder perturbation potential, a difference between the current value at the peak and the current value at the valley of the stepladder perturbation potential, sensitive currents at the stepladder perturbation potential between uphill and downhill, sensitive currents at a start point and an end point of the cycle of each stepladder perturbation potential, one of the average values of the sensitive currents obtained from the stepladder perturbation potential, or values that can be obtained by expressing the current values obtained from this with mathematical functions such as four arithmetic operations, exponential, logarithmic, trigonometric functions.

3. The bioanalyte concentration measurement method of claim 1, wherein the second sensitive current is obtained within 0.1 to 1 s after obtaining the first sensitive current.

4. The bioanalyte concentration measurement method of claim 1, further comprising:
adjusting weights between neurons present in several layers in the artificial neural network; and the artificial neural network automatically extracting features using one of convolutional neural networks (CNN), deep belief networks (DBN), and recurrent neural networks (RNNs) according to a structure.

5. The bioanalyte concentration measurement method of claim 1, wherein the feature automatic extraction uses restricted Boltzmann machines (RBM), and includes optimizing in which distribution of the input data and distribution of reconstructed data determined (stochastic decision) according to a probability are similar.

6. The bioanalyte concentration measurement method of claim 5, wherein the optimizing includes determining a weight and a bias value of the entire artificial neural network, and using an activation function.

7. The bioanalyte concentration measurement method of claim 6, wherein the artificial neural network is used for a classifier that classifies a type of data through a change in the activation function or structure of the output layer, or for regression that estimates a value.

## Patentansprüche

1. Ein Verfahren zur Messung der Konzentration von Bioanalyten unter Verwendung von mehrschichtigem Lernen mit künstlicher Intelligenz, wobei das Verfahren die folgenden Schritte aufweist:
Injizieren einer flüssigen biologischen Probe in eine Probenzelle, die mit einer Arbeitselektrode und einer Hilfselektrode eines elektrochemischen Biosensorstreifens ausgestattet ist, der in eine Vorrichtung zur Messung biometrischer Informationen (S 120) aufgenommen ist, in der ein Elektronentransportmedium und ein Redox-Enzym fixiert sind, welches in der Lage ist, eine Redoxreaktion eines Analyten zu katalysieren;
Erhalten, zu mindestens einem Zeitpunkt, eines ersten Sensitivstroms an einem charakteristischen Punkt durch Anlegen einer konstanten Gleichspannung an die Arbeitselektrode, um die Redoxreaktion des Analyten zu initiieren und um mit einer Elektronentransferreaktion fortzufahren (S 130);
Erhalten, zu zwei oder mehr Zeitpunkten, eines zweiten Sensitivstroms durch Anlegen eines Störpotentials vom Stufenleiter-Typ A - nach Anlegen der konstanten Gleichspannung (S 140);
Berechnen eines eine Konzentration des Analyten charakterisierenden Merkmals aus dem ersten Sensitivstrom oder dem zweiten Sensitivstrom; und
Korrigieren der berechneten Konzentration des Analyten unter Verwendung von mehrschichtigem Lernen mit künstlicher Intelligenz, so dass der Einfluss von zumindest zwei störenden Spezies in der biologischen Probe minimiert wird,
wobei das Korrigieren der berechneten Konzentration des Analyten das Berechnen eines neuen, eine Konzentration des Analyten charakterisierenden Merkmals durch erneutes Erfassen des ersten und zweiten Sensitivstroms durch mehrschichtiges Lernen mit künstlicher Intelligenz umfasst, und
wobei mehrschichtiges Lernen mit künstlicher Intelligenz beinhaltet:
Erstellen eines Algorithmus mehrschichtigen Lernens mit einem künstlichen neuronalen Netzwerk durch:
Gewinnen von Blutproben unter verschiedenen experimentellen Bedingungen und Gewinnen von Lerndaten durch wiederholte Messungen, wobei die erhaltenen, eindimensionale Zeitreihendaten darstellenden Lerndaten eine elektrochemische Reaktion des Analyten über die Zeit darstellen;
Konvertieren der erhaltenen Lerndaten in eine bestimmte Verteilung durch Normalisierung oder Standardisierung der Lerndaten, wobei die konvertierten Daten durch Kombinieren von Mehrkanaldaten oder Ausführen einer Domänenkonvertierung signalverarbeitet werden; und
Lernen des Algorithmus anhand der konvertierten Lerndaten, wobei der Algorithmus so ausgebildet ist, dass er ein Ergebnis in Abhängigkeit von einer Eingabe unter Verwendung der Technik des mehrschichtigen Lernens mit einem künstlichen neuronalen Netz ausgibt;
wobei das mehrschichtige Lernen mit künstlicher Intelligenz die zumindest zwei störenden Spezies, welche keinen Hämatokrit darstellen, korrigiert; und
wobei die Konzentrationsmessung des Analyten in der biologischen Probe durch mehrschichtiges Lernen mit künstlicher Intelligenz so korrigiert wird, dass keine Wartezeit für einen Temperaturausgleich erforderlich ist.

2. Das Verfahren zur Messung der Konzentration von Bioanalyten nach Anspruch 1, bei dem ein Merkmal mit einem Merkmalspunkt erzeugt wird, indem ein Merkmalspunkt ausgewählt wird, der in den ersten oder zweiten Sensitivströmen eine unterschiedliche lineare Abhängigkeit von dem Analyten und einem störenden Material aufweist,
ein Merkmal aus dem Merkmalspunkt konstruiert wird, und
eine aus dem obigen Merkmal durch das Lernen mit künstlicher Intelligenz zusammengesetzte Testformel erzeugt wird, auf der Grundlage des zweite Sensitivstroms in der Nähe einer Spitzen- und Talspannung einer spezifischen Stufenleiter,
der Krümmung der aus den Sensitivströmen jeder Stufe in einem Stufenleiter-Störpotential bestehenden Kurve,
der Differenz zwischen dem Stromwert am Scheitelpunkt und dem Stromwert am Talpunkt des Stufenleiter-Störpotentials,
von Sensitivströmen auf dem Stufenleiter-Störpotential zwischen Anstieg und Abfall,
von Sensitivströmen an einem Startpunkt und einem Endpunkt des Zyklus jedes Stufenleiter-Störpotentials,
eines der aus dem Stufenleiter-Störpotenzial gewonnenen Mittelwerte der Sensitivströme,
oder von Werten, die durch Beschreiben der gewonnenen Stromwerte mittels mathematischer Funktionen, wie den vier arithmetischen Operationen, exponentiellen, logarithmischen, trigonometrischen Funktionen, erhalten werden können.

3. Das Verfahren zur Messung der Konzentration von Bioanalyten nach Anspruch 1, wobei der zweite Sensitivstrom innerhalb von 0,1 bis 1 Sekunden nach Erhalt des ersten Sensitivstroms erhalten wird.

4. Das Verfahren zur Messung der Konzentration von Bioanalyten nach Anspruch 1, ferner aufweisend das
Einstellen von Gewichtungen zwischen in dem künstlichen neuronalen Netz in mehreren Schichten vorhandenen Neuronen, wobei das künstliche neuronale Netz automatisch Merkmale extrahiert, indem es anhand einer Struktur eines der folgenden Netze verwendet: Faltende neuronale Netze (CNN), Deep Belief Netze (DBN) und rekurrente neuronale Netze (RNN).

5. Das Verfahren zur Messung der Konzentration von Bioanalyten nach Anspruch 1, wobei die automatische Merkmalsextraktion eingeschränkte Boltzmann-Maschinen (RBM) verwendet und eine Optimierung beinhaltet, bei der die Verteilung der Eingabedaten und die Verteilung der rekonstruierten Daten, die gemäß einer Wahrscheinlichkeit bestimmt werden (stochastische Entscheidung), ähnlich sind.

6. Das Verfahren zur Messung der Konzentration von Bioanalyten nach Anspruch 5, wobei die Optimierung die Bestimmung einer Gewichtung und eines Vorspannungs-Wertes des gesamten künstlichen neuronalen Netzes und die Verwendung einer Aktivierungsfunktion umfasst.

7. Das Verfahren zur Messung der Konzentration von Bioanalyten nach Anspruch 6, wobei das künstliche neuronale Netz für einen Klassifikator verwendet wird, der durch eine Änderung der Aktivierungsfunktion oder der Struktur der Ausgabeschicht einen Datentyp klassifiziert, oder für eine Regression, die einen Wert schätzt.

## Revendications

1. Procédé de mesure de la concentration d'un bioanalyte à l'aide d'un apprentissage profond à intelligence artificielle, le procédé comprenant :
l'injection d'un échantillon biologique liquide dans une cellule d'échantillon équipée d'une électrode de travail et d'une électrode auxiliaire d'une bande de biocapteur électrochimique chargée dans un dispositif de mesure d'informations biométriques (S120), dans laquelle un milieu de transport d'électrons et une enzyme redox qui est capable de catalyser une réaction redox d'un analyte sont fixés ;
l'obtention d'un premier courant sensible à un point caractéristique d'au moins un instant par l'application d'une tension CC constante à l'électrode de travail pour initier la réaction redox de l'analyte et pour poursuivre par une réaction de transfert d'électrons (S130) ;
l'obtention d'un deuxième courant sensible à au moins deux instants par l'application d'un potentiel de perturbation de type en escabeau en Λ après l'application de la tension CC constante (S140) ;
le calcul d'une caractéristique représentant une concentration de l'analyte à partir du premier courant sensible ou du deuxième courant sensible ; et
la correction de la concentration de l'analyte calculée par l'utilisation d'un apprentissage profond à intelligence artificielle de telle sorte qu'une influence d'au moins deux espèces interférentes dans l'échantillon biologique soit réduite au maximum,
dans lequel la correction de la concentration calculée de l'analyte comporte le calcul d'une nouvelle caractéristique représentant une concentration de l'analyte par la réacquisition des premier et deuxième courants sensibles par un apprentissage profond à intelligence artificielle, et
dans lequel l'apprentissage profond à intelligence artificielle comporte :
la réalisation d'un algorithme d'apprentissage profond de réseau neuronal artificiel par :
l'obtention d'échantillons de sang de diverses conditions expérimentales, et l'obtention de données d'apprentissage par l'intermédiaire de mesures répétées,
les données d'apprentissage obtenues, qui sont des données de série chronologique unidimensionnelles, représentant une réaction électrochimique de l'analyte dans le temps ;
la conversion des données d'apprentissage obtenues en une certaine distribution par la normalisation ou la standardisation des données d'apprentissage, les données converties étant traitées en signal par la combinaison de données multicanal ou la mise en oeuvre d'une conversion de domaine ; et
l'apprentissage de l'algorithme avec les données d'apprentissage converties, dans lequel l'algorithme est configuré pour émettre en sortie un résultat en fonction d'une entrée à l'aide d'une technique d'apprentissage profond de réseau neuronal artificiel ;
dans lequel l'apprentissage profond à intelligence artificielle corrige les au moins deux espèces interférentes autres que l'hématocrite ; et
dans lequel la mesure de concentration de l'analyte dans l'échantillon biologique est corrigée par un apprentissage profond à intelligence artificielle sans temps d'attente pour un équilibrage de température.

2. Procédé de mesure de la concentration d'un bioanalyte selon la revendication 1, qui est un procédé de réalisation d'une caractéristique avec un point caractéristique par la sélection d'un point caractéristique ayant une dépendance linéaire différente sur l'analyte et un matériau interférant dans les premier ou deuxième courants sensibles, en construisant une caractéristique à partir du point caractéristique, la création d'une formule de test composée de la caractéristique précitée par l'apprentissage par intelligence artificielle utilisant le deuxième courant sensible à proximité d'une tension de crête et de creux d'une échelle à échelonnement spécifique, une courbure de la courbe constituée des courants sensibles de chaque échelon dans un potentiel de perturbation en escabeau, une différence entre la valeur de courant au pic et la valeur de courant au creux du potentiel de perturbation en escabeau, des courants sensibles au potentiel de perturbation en escabeau entre une montée et une descente, des courants sensibles à un point de départ et un point final du cycle de chaque potentiel de perturbation en escabeau, l'une des valeurs moyennes des courants sensibles obtenus à partir du potentiel de perturbation en escabeau, ou des valeurs qui peuvent être obtenues en exprimant les valeurs de courant obtenues à partir de ceux-ci avec des fonctions mathématiques telles que les quatre opérations arithmétiques, des fonctions exponentielles, logarithmiques, trigonométriques.

3. Procédé de mesure de la concentration d'un bioanalyte selon la revendication 1, dans lequel le deuxième courant sensible est obtenu entre 0,1 et 1 s après l'obtention du premier courant sensible.

4. Procédé de mesure de la concentration d'un bioanalyte selon la revendication 1, comprenant en outre :
l'ajustement des poids entre des neurones présents dans plusieurs couches dans le réseau neuronal artificiel ; et l'extraction automatique des caractéristiques par le réseau neuronal artificiel à l'aide de réseaux neuronaux convolutifs (CNN), de réseaux de croyances profondes (DBN) ou de réseaux neuronaux récurrents (RNN) selon une structure.

5. Procédé de mesure de la concentration d'un bioanalyte selon la revendication 1, dans lequel l'extraction automatique de caractéristiques utilise des machines de Boltzmann restreintes (RBM), et comporte l'optimisation dans laquelle une distribution des données d'entrée et une distribution de données reconstruites déterminées (décision stochastique) selon une probabilité sont similaires.

6. Procédé de mesure de la concentration d'un bioanalyte selon la revendication 5, dans lequel l'optimisation comporte la détermination d'un poids et d'une valeur de biais de l'ensemble du réseau neuronal artificiel, et l'utilisation d'une fonction d'activation.

7. Procédé de mesure de la concentration d'un bioanalyte selon la revendication 6, dans lequel le réseau neuronal artificiel est utilisé pour un classificateur qui classifie un type de données par l'intermédiaire d'un changement de la fonction ou de la structure d'activation de la couche de sortie, ou pour une régression qui estime une valeur.
